# EUROPEAN PATENT APPLICATION

(11) **EP 4 803 901 A1**
(43) Date of publication of application: **09.09.2026**
(21) Application number: 24885241.0
(22) Date of filing: 08.08.2024
(51) Int. Cl.: G01N 33/493, G01N 33/483

(54) **IMAGING METHOD AND IMAGING PROGRAM**

(30) Priority: 31.10.2023 JP 2023186985
(71) Applicant: ARKRAY, Inc., Kyoto-shi, Kyoto 601-8045 (JP)
(72) Inventor: FUJIMOTO, Koji, Kyoto-shi, Kyoto 602-0008 (JP)
(74) Representative: MFG Patentanwälte Meyer-Wildhagen Meggle-Freund Gerhard PartG mbB
(86) International application number: PCT/JP2024/028555
(87) International publication number: WO 2025/094464

(57) **Abstract**

A urine material component analyzer: moves, along a moving plane intersecting the direction of gravity, an imaging location of an imaging device that images, along the direction of gravity, a cell having a hollow portion in which a urine sample is enclosed and acquires, at a plurality of imaging locations, reference height information representing a position, in the direction of gravity in the cell, of material components included in the urine sample when the material components are in focus and reference imaging location information representing imaging locations of the material components imaged by the imaging device using coordinate values of two-dimensional coordinates provided in the moving plane; calculates, by interpolation using the reference height information and the reference imaging location information at the plurality of imaging locations, positions of the material components at other imaging locations different from the plurality of imaging locations; and controls the imaging device to focus on the calculated positions of the material components and capture sample images that are images of the urine sample at the other imaging locations.

## Description

### TECHNICAL FIELD

This disclosure relates to an imaging method and an imaging program for imaging material components included in samples.

### BACKGROUND ART

Non-patent document 1 describes a microscopic examination in which a clinical laboratory technician places a urine sample on a microscope slide and visually inspects material components included in the urine sample with a microscope.

### <Non-Patent Document 1>

Non-patent Document 1: Japanese Journal of Medical Technology, Vol. 66 (2017), J-STAGE-1, pp. 18-50, Japanese Association of Medical Technologists

### SUMMARY OF INVENTION

### Technical Problem

When performing a urine sediment examination with a testing device, the clinical laboratory technician uses an imaging device to image material components included in the urine sample instead of visually inspecting material components in the urine sample with a microscope.

However, since the size of material components is on the order of µm, it is difficult to focus the imaging device on the material components, and since the clinical laboratory technician does not know where material components are located, it is also difficult to focus on a preset subject distance and then capture images. Consequently, the clinical laboratory technician may repeatedly perform control to focus on the material components through trial and error for each imaging location.

It is an object of this disclosure to provide an imaging method and an imaging program that, compared with focusing on material components included in a urine sample and capturing an image at each imaging location of a cell in which the urine sample is enclosed, can shorten the amount of time required to image the material components.

### Solution to Problem

In order to achieve the above object, as for an imaging method pertaining to an aspect of this disclosure, a computer executes a process to: move, along a moving plane intersecting the direction of gravity, an imaging location of an imaging device that images, along the direction of gravity, a cell having a hollow portion in which a urine sample is enclosed and acquire, at a plurality of imaging locations, reference height information representing a position, in the direction of gravity in the cell, of material components included in the urine sample when the material components are in focus and reference imaging location information representing imaging locations of the material components imaged by the imaging device using coordinate values of two-dimensional coordinates provided in the moving plane; calculate, by interpolation using the reference height information and the reference imaging location information at the plurality of imaging locations, positions of the material components at other imaging locations different from the plurality of imaging locations; and control the imaging device to focus on the calculated positions of the material components and capture sample images that are images of the urine sample at the other imaging locations.

Moreover, in order to achieve the above object, an imaging program pertaining to an aspect of this disclosure is a program for causing a computer to execute a process to: move, along a moving plane intersecting the direction of gravity, an imaging location of an imaging device that images, along the direction of gravity, a cell having a hollow portion in which a urine sample is enclosed and acquire, at a plurality of imaging locations, reference height information representing a position, in the direction of gravity in the cell, of material components included in the urine sample when the material components are in focus and reference imaging location information representing imaging locations of the material components imaged by the imaging device using coordinate values of two-dimensional coordinates provided in the moving plane; calculate, by interpolation using the reference height information and the reference imaging location information at the plurality of imaging locations, positions of the material components at other imaging locations different from the plurality of imaging locations; and control the imaging device to focus on the calculated positions of the material components and capture sample images that are images of the urine sample at the other imaging locations.

### Advantageous Effects of Invention

According to this disclosure, there is the advantageous effect that, compared with focusing on material components included in a urine sample and capturing an image at each imaging location of a cell in which the urine sample is enclosed, the amount of time required to image the material components can be shortened.

### BRIEF DESCRIPTION OF DRAWINGS

FIG. 1 is a drawing showing an example configuration of a medical information processing system.
FIG. 2 is a drawing showing an example device configuration of a urine material component analyzer.
FIG. 3 is a drawing showing an example of a cell.
FIG. 4 is a drawing showing an example arrangement of the cell on a plate.
FIG. 5 is a drawing showing another example arrangement of the cell on the plate.
FIG. 6 is a drawing showing example functional configurations of the urine material component analyzer.
FIG. 7 is a flowchart showing an example of the flow of an imaging process for imaging material components included in a urine sample.
FIG. 8 is a drawing showing an example of imaging conditions when imaging material components.
FIG. 9 is a drawing showing an example of an imaging plane.
FIG. 10 is a drawing showing an example when the imaging plane is viewed along the Z-axis direction.
FIG. 11 is a drawing showing an example of an imaging plane passing through reference points at each of reference imaging locations.
FIG. 12 is a flowchart showing an example modification of the imaging process for imaging material components included in a urine sample.
FIG. 13 is a drawing showing an example of panoramic image generation.
FIG. 14 is a drawing showing an example of a panoramic image configured by side edge portions of images.

### DESCRIPTION OF EMBODIMENT

An embodiment will be described below with reference to the drawings. It will be noted that the same reference signs are assigned to the same components and the same processes throughout the drawings, and redundant description will be omitted. Dimensional ratios in the drawings are exaggerated for convenience of description and may differ from actual ratios.

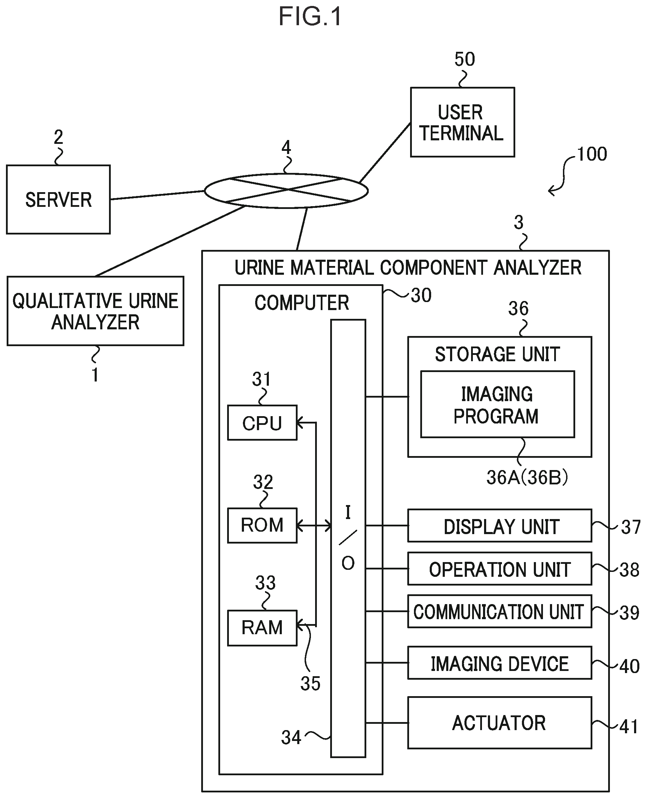

FIG. 1 is a drawing showing an example configuration of a medical information processing system 100 in a urine sediment examination. A urine sediment examination is an examination that identifies types of material components included in a patient's urine sample from the shapes of the material components, for example, and performs various types of analyses such as the numbers and concentrations of the material components. In a urine sediment examination, types of material components are identified, such as, for example, red blood cells, white blood cells, non-squamous epithelial cells, squamous epithelial cells, bacteria (also called "bacteria"), crystals, yeasts, hyaline casts, other casts (also called pathological casts), mucus threads, sperm, and white blood cell clumps.

As shown in FIG. 1, the medical information processing system 100 includes a qualitative urine analyzer 1, a server 2, a urine material component analyzer 3, and a user terminal 50, and the qualitative urine analyzer 1, the server 2, the urine material component analyzer 3, and the user terminal 50 are connected to each other by a communication line 4. There are no restrictions on the mode of connection by the communication line 4, and it may be wired or wireless. Furthermore, there are also no restrictions on the type of the communication line 4, and a line such as, for example, the internet, a local area network (LAN), or a wide area network (WAN) can be used for the communication line 4.

When a urine sediment examination is performed, a qualitative urinalysis is performed beforehand using the qualitative urine analyzer 1. A "qualitative urinalysis" is a test in which, for example, urine is applied to a test paper called Tes-Tape, which reacts with components to be measured in the urine sample and changes color, and the change in color is measured to determine whether the components to be measured are present in the urine sample. Furthermore, a qualitative urinalysis also measures the concentrations of the components to be measured in the urine sample. A qualitative urinalysis measures, for example, the pH of the urine sample, the specific gravity of the urine sample, and the turbidity of the urine sample, as well as the presence and content of protein, sugar, ketone bodies, bilirubin, urobilinogen, occult blood reactions, nitrites, and white blood cells in the urine sample.

The qualitative urine analyzer 1 includes a barcode reader (not shown in the drawings) for reading the sample ID of a urine sample to be measured from a barcode label stuck to the side of a tube (not shown in the drawings) containing the urine sample, and the qualitative urine analyzer 1 associates the qualitative urinalysis results of the urine sample it has measured with the sample ID of the urine sample and sends the qualitative urinalysis results associated with the sample ID through the communication line 4 to the server 2.

When the server 2 receives the qualitative urinalysis results associated with the sample ID from the qualitative urine analyzer 1, it stores the qualitative urinalysis results associated with the sample ID in a storage device.

The qualitative urine analyzer 1 does not invariably need to store the qualitative urinalysis results in the server 2 and may store them in a storage device of the qualitative urine analyzer 1. In this case, the server 2 becomes unnecessary in the medical information processing system 100.

It will be noted that some items in urine sample testing, such as determining the presence of bacteria like E. coli, enterococci, staphylococci, and streptococci, are difficult to analyze with just a qualitative urinalysis. Consequently, the clinical laboratory technician sends the sample ID of a urine sample that has finished being qualitatively analyzed by the qualitative urine analyzer 1 through the communication line 4 from the qualitative urine analyzer 1 to the urine material component analyzer 3 and requests the urine material component analyzer 3 to image the material components included in the urine sample.

Upon receiving the sample ID from the qualitative urine analyzer 1, the urine material component analyzer 3 images the material components included in the urine sample and sends the images of the material components it has captured to the user terminal 50. It will be noted that "image" in this embodiment is an example of a sample image of a urine sample and may be either a still image or video.

The user terminal 50 is a terminal used by a clinical laboratory technician who specializes in classifying material components included in urine samples (hereinafter called a "specialized clinical laboratory technician"). The specialized clinical laboratory technician classifies material components included in urine samples by referring to images of the urine samples received from the urine material component analyzer 3.

As shown in FIG. 1, the urine material component analyzer 3 is configured using a computer 30, for example. The computer 30 includes a central processing unit (CPU) 31, which is an example of a processor, a read-only memory (ROM) 32, a randam-access memory (RAM) 33, and an input/output interface (I/O) 34, and the CPU 31, the ROM 32, the RAM 33, and the I/O 34 are connected to each other by a bus 35.

The ROM 32 stores, for example, a startup program (Basic Input Output System: BIOS) for the CPU 31 to perform a process for starting up the computer 30. Furthermore, the RAM 33 is used as a temporary workspace by the CPU 31.

The computer 30 configuring the urine material component analyzer 3 may be a personal computer (PC) or a portable device such as a smartphone or a tablet computer, for example.

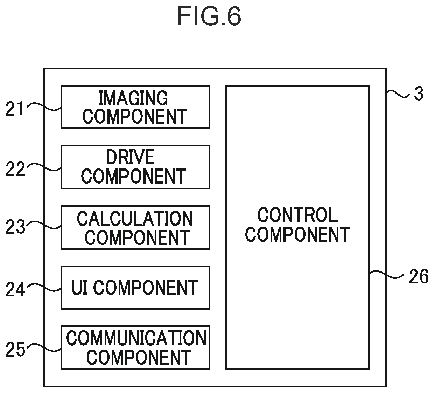

The CPU 31, the ROM 32, the RAM 33, and the I/O 34 configure a control component 26 (see FIG. 6) described below.

Connected to the I/O 34 are a storage unit 36, a display unit 37, an operation unit 38, a communication unit 39, an imaging device 40, and an actuator 41. Each of these units is connected through the I/O 34 to the CPU 31.

The storage unit 36 is an example of a storage device that maintains stored information even if the power supplied to the storage unit 36 is cut off, and a semiconductor memory such as a solid-state drive (SSD), for example, is used, but a hard disk may also be used. Furthermore, the storage unit 36 may be a portable semiconductor memory removable from the computer 30, such as a universal serial bus (USB) memory or a memory card, for example.

Imaging programs 36A, 36B that the CPU 31 reads in order to capture images of material components included in urine samples are stored in advance in the storage unit 36. Furthermore, in addition to the imaging programs 36A, 36B, various types of parameters that the CPU 31 references when controlling the urine material component analyzer 3 are also stored in advance in the storage unit 36. It will be noted that it is not invariably necessary for the imaging programs 36A, 36B and the various types of parameters to be stored in the storage unit 36, and they may be stored in the ROM 32.

For the display unit 37, a liquid crystal display (LCD) or an organic electroluminescence (EL) display, for example, may be used. The display unit 37 may have an integrated touch panel. The display unit 37 displays, for example, results of processing executed in accordance with instructions received from the clinical laboratory technician, captured images, and notifications about the processing.

The operation unit 38 is provided with devices for inputting operations, such as buttons, a touch panel, a keyboard, a mouse, and a pointing device, for example. The clinical laboratory technician issues instructions to the CPU 31 of the urine material component analyzer 3 by operating the operation unit 38.

The communication unit 39 is connected to the communication line 4 and has a communication protocol for communicating data with the qualitative urine analyzer 1, the server 2, and the user terminal 50.

The imaging device 40 includes an imaging lens and an image sensor disposed on the optical axis of the imaging lens and images material components included in urine samples. The image sensor is, for example, a charge-coupled device (CCD). The images captured by the imaging device 40 may be either grayscale images or color images, but as an example it will be assumed that the imaging device 40 captures color images.

The actuator 41 moves the imaging location of the urine sample imaged by the imaging device 40. It will be noted that the operations of the imaging device 40 and the actuator 41 will be described in detail further below.

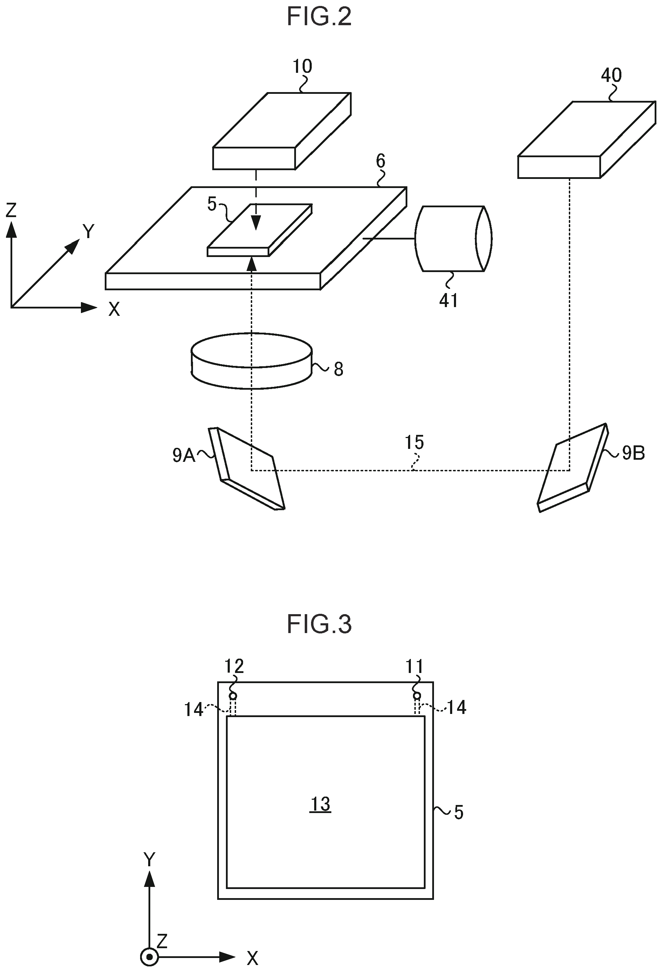

Next, the principle by which a urine sample is imaged in the urine material component analyzer 3 will be described. FIG. 2 is a drawing showing an example device configuration of the urine material component analyzer 3. The Z-axis in FIG. 2represents a vertical direction (i.e., the direction of gravity), the X-axis represents a direction perpendicular to the Z-axis, and the Y-axis represents a direction perpendicular to the X-axis and the Z-axis. That is, the X-axis and the Y-axis define two-dimensional coordinates that represent the position of a plane (referred to as an "XY plane") perpendicular to the Z-axis, and the X-axis, the Y-axis, and the Z-axis define three-dimensional coordinates of the space where the urine material component analyzer 3 is. The distance along the Z-axis will be referred to as "height."

The urine material component analyzer 3 includes a plate 6, the imaging device 40, a lens 8, a mirror 9A, a mirror 9B, a light source 10, and the actuator 41, and on the plate 6 is disposed a cell 5 in which a urine sample corresponding to a sample ID received from the qualitative urine analyzer 1 is enclosed.

The cell 5 is configured by a transparent container formed of a synthetic resin, such as acrylic, polycarbonate, or polyethylene terephthalate, for example, to allow the imaging device 40 to capture images of material components included in the enclosed urine sample.

FIG. 3 is a drawing showing an example of the cell 5. As shown inFIG. 3, inside the cell 5 is a hollow portion 13 that can enclose a urine sample, and when the clinical laboratory technician uses a pipette or the like to flow the urine sample into an inlet 11 provided in the cell 5, the urine sample reaches the hollow portion 13 through a flow path 14. The cell 5 has an outlet 12 in addition to the inlet 11, and the urine sample overflowing due to inflow into the hollow portion 13 is discharged with a flow path 14 from the outlet 12. It will be noted that the cell 5 being "transparent" means that, when an object located in back of the cell 5 or an object located in the hollow portion 13 of the cell 5 is viewed through the cell 5, the cell 5 has a degree of transparency that allows the shape of that object to be checked.

The cell 5 is disposed on the plate 6 so that the lower surface of the cell 5, which is the surface that contacts the plate 6, is horizontal (parallel to the XY plane). "Parallel" in this embodiment refers to a state in which the lower surface of the cell 5 and the XY plane do not intersect no matter how far they are extended, i.e., a completely parallel state, and also includes a case in which the two surfaces are inclined to an extent that they may be considered parallel.

Although there are no restrictions on the shape of the cell 5 when the cell 5 is viewed in the direction of the XY plane (as viewed in a plan view from the Z-direction), in this embodiment, as an example, the cell 5 is rectangular. In this case, the X-axis is set along one side of the cell 5, and the Y-axis is set along a side perpendicular to the one side of the cell along the X-axis.

It will be noted that the length of the cell 5 along the Z-axis direction, i.e., the height of the cell 5 is the same at every point, and the height of the cell 5 is shorter than the lengths of the cell 5 along the X-axis direction and the Y-axis direction. That is, the cell 5 has the shape of a rectangular cuboid.

The light source 10 is, for example, provided in a position that is higher along the Z-axis direction than the position of the cell 5 placed on the plate 6 and opposes the cell 5, and the light source 10 emits light toward the cell 5 to allow the imaging device 40 to easily capture images of the material components included in the urine sample.

The imaging device 40 images the material components included in the urine sample enclosed in the cell 5. The imaging device 40 is, for example, disposed on the upper surface of a housing (not shown in the drawings) of the urine material component analyzer 3 so that an imaging lens included in the imaging device 40 faces downward along the Z-axis direction. In this case, an optical axis 15 of the imaging device 40, i.e., the optical axis of the imaging lens, also faces downward along the Z-axis direction, so the imaging device 40 images the mirror surface of the mirror 9B located on an extension line of the optical axis (see FIG. 2). However, by adjusting the angle of the mirror 9A so that the cell 5 appears on the mirror surface of mirror 9B, the imaging device 40 can image the material components included in the urine sample enclosed in the cell 5.

That is, the imaging device 40 images the cell 5 from the bottom up along the Z-axis direction from a position opposing the lower surface of the cell 5. In other words, the imaging device 40 images the urine sample along the height direction of the cell 5.

The material components included in the urine sample enclosed in the cell 5 settle toward the lower surface of the cell 5 over time due to the effect of gravity. Consequently, the imaging device 40 more easily images the material components included in the urine sample compared with imaging the cell 5 from the surface of the cell 5 that one sees when the cell 5 disposed on the plate 6 is viewed from top down along the Z-axis direction, i.e., the upper surface of the cell 5.

It will be noted that the higher the height of the cell 5 is, the more likely it is that the material components included in the urine sample will be imaged in a state in which they overlie each other, and the wider the focus adjustment range of the imaging device 40 will be, so it is preferred that the height of the cell 5 be 1000 µm.

The lens 8 is disposed between the cell 5 and mirror 9A so that the lens 8 intersects the optical axis 15 of the imaging device 40, i.e., so that the optical axis of the lens 8 coincides with the optical axis 15 of the imaging device 40, and magnifies the material components included in the urine sample to a magnification ratio designated by the clinical laboratory technician. Consequently, the imaging device 40 can image the material components included in the urine sample at the magnification ratio designated by the clinical laboratory technician. Naturally, when the imaging device 40 has an image enlarging/reducing function (what is called a zoom function), the enlarging/reducing function of the imaging device 40 may be used to image the material components included in the urine sample at the magnification ratio designated by the clinical laboratory technician.

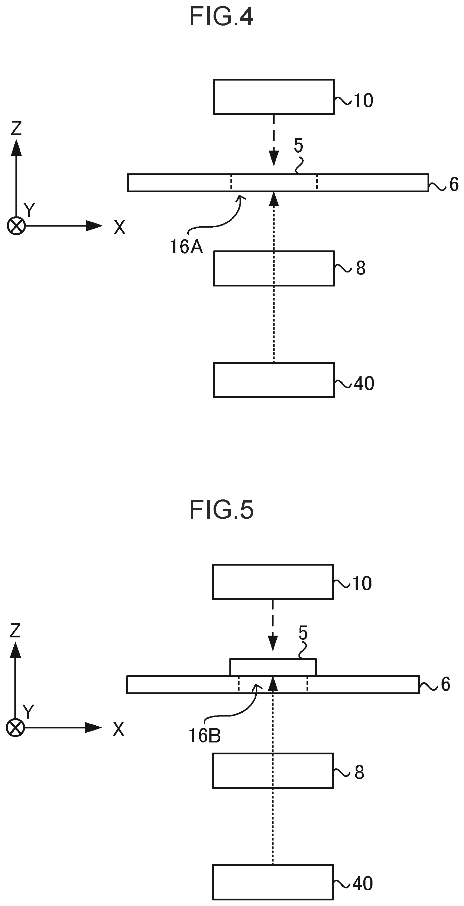

It will be noted that the portion of the plate 6 on which the lower surface of the cell 5 rests when the cell 5 is disposed on the plate 6 is, like the cell 5, configured using a transparent material so as not to hinder the imaging of the cell 5 by the imaging device 40. Furthermore, as shown in FIG. 4, the cell 5 may be embedded in a hole 16A in the plate 6 formed to match the shape of the cell 5, or, as shown in FIG. 5, the cell 5 may be placed over a hole 16B that penetrates the plate. There are no restrictions on the shape of the hole 16B in this case.

Furthermore, in the example device configuration of the urine material component analyzer 3 shown in FIG. 2, an example is shown where the imaging device 40 is disposed with the imaging lens facing downward along the Z-axis direction. However, as shown in FIG. 4 and FIG. 5, the imaging device 40 may be disposed under the cell 5 with the imaging lens facing upward along the Z-axis direction. In this case, the mirrors 9A, 9B that bend the optical axis 15 become unnecessary.

That is, there are no restrictions on the position of the imaging device 40 in the urine material component analyzer 3, and the imaging device 40 may be mounted in any position. By adjusting the positions and numbers of the mirrors 9A, 9B, for example, the urine material component analyzer 3 can image the material components included in the urine sample.

The actuator 41 is a drive source that moves the plate 6 independently in the X-axis direction and the Y-axis direction. Specifically, the actuator 41 includes a pulse motor, which rotates an amount according to the number of applied pulses, and a conversion mechanism, such as a rack-and-pinion mechanism and a ball screw, which converts the rotation of the pulse motor into movement of the plate 6 in the X-axis direction and the Y-axis direction. The actuator 41 moves the plate 6 a distance according to the number of pulses applied to the pulse motor. Consequently, by driving the actuator 41, the imaging location of the cell 5 can be moved along the XY plane while the position of the imaging device 40 and the positions of the mirrors 9A, 9B remain fixed. In this way, since the imaging location of the imaging device 40 moves along the XY plane, hereinafter the XY plane will be referred to as a "moving plane."

It will be noted that the imaging device 40 may be any device with which it is possible to select between capturing still images and video, such as, for example, a digital camera, a camera built into a smartphone, a camera built into a wearable device, or a camera built into the computer 30. The urine material component analyzer 3 pertaining to this embodiment images material components included in urine samples using a camera built into a smartphone.

Next, functions of the urine material component analyzer 3 will be described. FIG. 6 is a drawing showing example functional configurations of the urine material component analyzer 3. As shown in FIG. 6, the urine material component analyzer 3 includes an imaging component 21, a drive component 22, a calculation component 23, a user interface (UI) component 24, a communication component 25, and a control component 26.

The imaging component 21 captures, with the imaging device 40, images of the material components included in the urine sample enclosed in the cell 5. The images of the material components captured by the imaging device 40 may be still images or video. When imaging the material components, the imaging component 21 inputs a number of pulses according to the intended moving distance of the imaging lens to the pulse motor of the imaging device 40 that moves the imaging lens a distance according to the number of applied pulses. Because of this, the imaging component 21 performs focus control to change the position of the imaging lens and focus on the position of a subject. The coordinate value z of the Z-coordinate of the position on which the imaging device 40 is focused will be referred to as a "focus position" in, for example, the space where there are subjects such as material components in the urine sample enclosed in the cell 5.

The focus position when the material components are in focus represents, for example, the height, in the cell 5, of the material components that are in focus. Consequently, height information representing the height of the material components is represented by the coordinate value z of the Z-axis.

The drive component 22 controls the actuator 41 to move the plate 6 along the moving plane to a designated position. When the plate 6 moves, the cell 5 disposed on the plate 6 also moves. Consequently, the drive component 22 moves the imaging location of the cell 5 imaged by the imaging device 40. Imaging location information representing the position of the imaging location of the imaging device 40, i.e., moving plane coordinate values, are given by the coordinate value x of the X coordinate and the coordinate value y of the Y coordinate in the moving plane, such as (x, y), for example.

The calculation component 23 acquires the moving plane coordinate values of imaging locations at at least three or more predetermined imaging locations and height information representing the positions of the material components in the cell 5 at each of the imaging locations.

If the height information and the moving plane coordinate values are known, the positions, in the cell 5, of material components that are in focus can be known. Consequently, the calculation component 23 generates an interpolation equation representing a plane obtained by interconnecting with straight lines the positions of material components that are in focus.

The interpolation equation is, for example, an equation that uses the moving plane coordinate values as explanatory variables and the height information as response variables. That is, if the imaging locations (i.e., the moving plane coordinate values) of the cell 5 imaged by the imaging device 40 are known, the height information of other imaging locations other than the at least three or more imaging locations (hereinafter called "reference imaging locations") that were used to generate the interpolation equation is calculated from the interpolation equation.

For convenience of description, the plane passing through the positions of the material components that are in focus at each of the reference imaging locations will be called an "imaging plane." Although in this embodiment the imaging plane is generated as a flat plane as an example, it may also be a curved plane.

At other imaging locations other than the reference imaging locations, the imaging component 21 focuses on the height of the imaging plane and captures images of the material components included in the urine sample, but details will be described further below. Hereinafter, the other imaging locations different from the reference imaging locations will be called "interpolated imaging locations."

The UI component 24 notifies the control component 26, described below, of instructions from the clinical laboratory technician received through the operation unit 38. Furthermore, the UI component 24 displays on the display unit 37 the images of the material components included in the urine sample captured by the imaging component 21, various information types of information processed by the control component 26 in accordance with instructions received from the clinical laboratory technician, and notifications about the processing.

The communication component 25 communicates data with the qualitative urine analyzer 1, the server 2, and the user terminal 50 through the communication unit 39.

The control component 26 controls processing in the imaging component 21, the drive component 22, the calculation component 23, the UI component 24, and the communication component 25, allowing the urine material component analyzer 3 to execute operations instructed by the clinical laboratory technician.

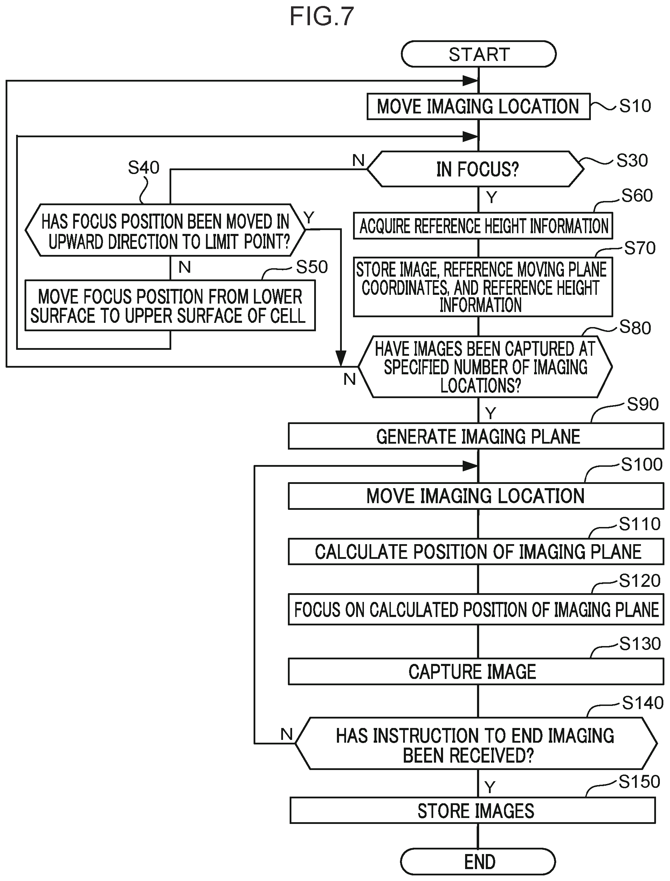

Next, the action of the urine material component analyzer 3 will be described in detail. FIG. 7 is a flowchart showing an example of the flow of an imaging process for imaging material components included in a urine sample, which is executed by the CPU 31 of the urine material component analyzer 3 when it receives the sample ID of the urine sample from the qualitative urine analyzer 1. The CPU 31 of the urine material component analyzer 3 reads the imaging program 36A stored in the storage unit 36 and executes the imaging process.

To make it easier to image the material components included in the urine sample, the control component 26 performs the imaging process to image the material components after a period of time elapses in which the material components are thought to naturally settle in the cell 5.

In step S10, the control component 26 for example moves the plate 6 along the X-axis and the Y-axis to a limit point where it cannot move the plate 6 any further. The moving plane coordinate values of the plate 6 at the limit point are set as the origin of the moving plane, i.e., (x, y) = (0, 0). The control component 26 controls the drive component 22 to move the plate 6 along the moving plane so that the intersection between the optical axis 15 of the imaging device 40 and the moving plane, i.e., the imaging location of the imaging device 40, comes within the range of the lower surface of the cell 5. For convenience of description, the destination imaging location will be called an "nth imaging location." n is a value representing the moving order to the imaging location and is expressed as an integer of 1 or more. Each time the control component 26 changes the imaging location, it adds 1 to n to identify the imaging location. The nth imaging location becomes a reference imaging location.

The moving plane coordinate values of the nth imaging location are stored in the storage unit 36 in advance, and the control component 26 acquires the moving plane coordinate values of the nth imaging location from the storage unit 36 and moves the plate 6 along the moving plane so that the imaging location of the imaging device 40 moves to the nth imaging location. Specifically, the control component 26 controls the drive component 22 to apply the same number of pulses as the coordinate value of the X-coordinate to the actuator 41 to move the plate 6 from the origin in the X-axis direction. Furthermore, the control component 26 controls the drive component 22 to apply the same number of pulses as the coordinate value of the Y-coordinate to the actuator 41 to move the plate 6 from the origin in the Y-axis direction. In this way, the moving plane coordinate values of the nth imaging location are expressed as coordinate values representing the distance from the origin of the moving plane along the X-axis and Y-axis. As a result, the plate 6 moves to the nth imaging location. It will be noted that the moving plane coordinate values of the nth imaging location will be referred to particularly as "reference moving plane coordinate values." The reference moving plane coordinate values are an example of reference imaging location information.

After moving the plate 6 to the nth imaging location, the control component 26 controls the imaging device 40 to move the focus position of the imaging device 40 to a limit point where it cannot be lowered any further in the downward direction, which is a direction from the upper surface to the lower surface of the cell 5. The height at the limit point is set as the origin of the Z-axis, i.e., z = 0.

It will be noted that when the height at the position of the lower surface of the cell 5 is known in advance, the control component 26 may focus the imaging device 40 on the height of the lower surface of the cell 5 and set the height corresponding to the position of the lower surface of the cell 5 as the origin of the Z-axis.

That is, the control component 26 aligns the focus position at the nth imaging location with the origin of the Z-axis.

In step S30, the control component 26 determines whether the imaging device 40 is focused on the material components in the urine sample enclosed in the cell 5. Specifically, the control component 26 converts the image that was captured by the imaging device 40 to grayscale, performs a convolution operation on the captured image using a Laplacian kernel of 3 pixels × 3 pixels, and calculates the variance of the operation results. Since the variance obtained from an image in focus tends to be large, the control component 26 determines that the material components are in focus at the current focus position when the variance calculated from the image is equal to or greater than a predetermined value. When the material components are not in focus, the control component 26 moves to step S40.

In step S40, the control component 26 determines whether the focus position of the imaging device 40 has been moved to a limit point where it cannot be raised any further in the upward direction, which is a direction from the lower surface to the upper surface of the cell 5. When the focus position has not been moved in the upward direction to the limit point, the control component 26 moves to step S50.

Since the material components are not in focus at the current focus position, in step 50, the control component 26 controls the imaging device 40 to move the focus position in the upward direction from the lower surface to the upper surface of the cell 5. Specifically, the control component 26 moves the focus position to a position corresponding to a value obtained by adding "1" to the coordinate value z. That is, the control component 26 applies one pulse to the pulse motor with which the imaging device 40 is equipped to move the focus position in the upward direction. If there are material components at the destination focus position, the imaging device 40 will focus on the material components.

Consequently, moving to step S30, the control component 26 repeatedly determines whether the material components are in focus. That is, the control component 26 moves the focus position in the upward direction one coordinate value at a time until the imaging device 40 focuses on the material components. It will be noted that the moving amount of the focus position is not limited to one coordinate value at a time and may, for example, be two coordinate values at a time.

When it is determined in the determination process of step S30 that the material components are in focus, the control component 26 moves to step S60.

In step S60, the control component 26 acquires the coordinate value z when it was determined that the material components are in focus as the height information of the material components at the nth imaging location, i.e., the reference height information. That is, the coordinate value z can also be said to be the number of pulses applied to the pulse motor of the imaging device 40 that is needed to move the imaging lens of the imaging device 40 from the position where the origin is in focus to a position where the material components are in focus.

In step S70, the control component 26 associates the reference moving plane coordinate values acquired in step S10 and the reference height information acquired in step S60 with the image that was captured focused on the height represented by the reference height information at the nth imaging location. The control component 26 stores the association between the reference moving plane coordinate values and the reference height information in the storage unit 36, for example.

In step S80, the control component 26 determines whether images of the material components have been captured at a predetermined specified number of imaging locations. As has already been described, the specified number of imaging locations is set to at least three or more.

When images of the material components have not been captured at the specified number of imaging locations, the control component 26 moves to step S10 and repeatedly executes the processes of steps S10 to S80 so that the specified number of images of the material components at the nth imaging locations are captured.

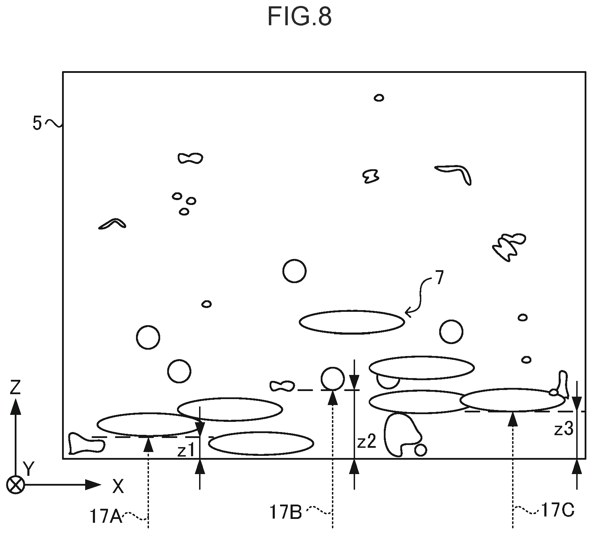

FIG. 8 is a drawing showing an example of imaging conditions when capturing images of material components at, for example, three reference imaging locations. In FIG. 8, arrow 17A indicates the first imaging location, arrow 17B indicates the second imaging location, and arrow 17C indicates the third imaging location. Furthermore, height z1, height z2, and height z3 indicate the reference height information at the first imaging location, the second imaging location, and the third imaging location, respectively, when the position of the lower surface of the cell 5 is set as the origin of the Z-axis.

It will be noted that when it is determined in the determination process of step S40 that the focus position of the imaging device 40 has been moved to the limit point in the upward direction, this means that there are no material components in the upward direction of the nth imaging location to which the imaging device 40 was moved by step S10. Consequently, in this case also, the control component 26 moves to step S10 to move the imaging location of the imaging device 40. If the control component 26 has moved to step S10 due to the determination process of step S40 resulting in a YES determination, an image focused on the material components is not captured at the nth imaging location prior to the control component 26 moving the imaging location. Consequently, if the control component 26 has moved to step S10 due to the determination process of step S40 resulting in a YES determination, the control component 26 does not add 1 to the value of n in step S10 but processes the next destination imaging location as the nth imaging location having the same value of n as the previous imaging location.

On the other hand, when it is determined by the determination process of step S80 that images of the material components have been captured at the specified number of imaging locations, the control component 26 moves to step S90.

In step S90, the control component 26 controls the calculation component 23 to generate n-number of positions represented by combinations of the reference moving plane coordinate values and the reference height information at the nth imaging location, i.e., a plane having the n-number of reference points as vertices. Hereinafter, the plane having n-number of reference points as vertices will be referred to as an "imaging plane 18."

The calculation component 23 interconnects each of the reference points with straight lines, for example, and generates, by linear interpolation on the reference points, a plane whose sides are the straight lines interconnecting the reference points. As has already been described, the calculation component 23 represents the plane using an interpolation equation that uses the moving plane coordinate values as explanatory variables and the height information as response variables.

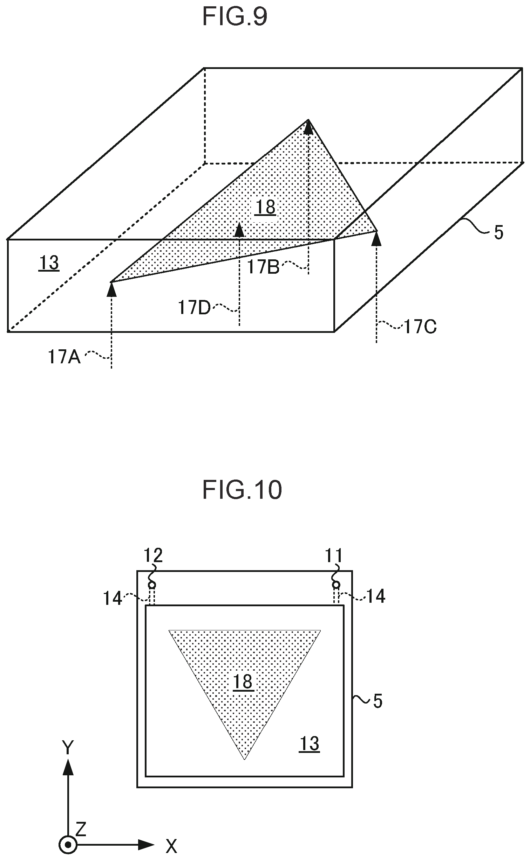

FIG. 9 is a drawing showing an example of the imaging plane 18 generated from the imaging conditions example shown in FIG. 8. When there are three reference imaging locations, the triangle generated by interconnecting the reference points at each of the reference imaging locations as shown in FIG. 9 becomes the imaging plane 18. The imaging plane 18 is a plane that contacts the material components at each of the reference imaging locations. Consequently, the probability that there will be material components in the position of the imaging plane 18 intersecting the optical axis 15 of the imaging device 40 at other imaging locations different from each of the reference imaging locations, i.e., interpolated imaging locations, is higher than the probability that there will be material components in positions located at different heights from the position of the imaging plane 18.

Consequently, at the interpolated imaging locations, the control component 26 captures images by focusing on the position of the imaging plane 18 intersecting the optical axis 15 without searching for positions where material components come into focus while shifting the focus position in the upward direction as with the reference imaging locations.

For that reason, in step S100, the control component 26 controls the drive component 22 to move the plate 6 along the moving plane and move the imaging location of the imaging device 40 to an interpolated imaging location. The control component 26 acquires the moving plane coordinate values at the destination interpolated imaging location. Arrow 17D in FIG. 9 indicates a destination interpolated imaging location. It will be noted that the interpolated imaging location is set within the range of a projection plane obtained by projecting the imaging plane 18 onto the moving plane.

In step S110, the calculation component 23 assigns the moving plane coordinate values at the destination interpolated imaging location to the interpolation equation for the imaging plane 18 generated in step S90 and calculates the position of the imaging plane 18 at the destination interpolated imaging location.

In step S120, the control component 26 controls the imaging device 40 to focus the imaging device 40 on the position of the imaging plane 18 calculated in step S110.

In step S130, the control component 26 controls the imaging device 40 to capture an image. Because of this, an image focused on the height where the most material components are thought to be present at the destination interpolated imaging location is captured. For convenience of description, the image captured by focusing on the position of the imaging plane 18 may be called the "imaging plane image."

In step S140, the control component 26 determines whether it has received an instruction to end imaging of the material components from the clinical laboratory technician through the operation unit 38. When it has not received an instruction to end imaging, the control component 26 moves to step S100, and it moves the imaging location of the imaging device 40 to a subsequent interpolated imaging location instructed by the clinical laboratory technician and captures an image focused on the imaging plane 18 at the new interpolated imaging location.

On the other hand, when the control component 26 has received an instruction to end imaging, it moves to step S150. In step S150, the control component 26 associates the moving plane coordinate values and height information at the imaging locations and the sample ID received from the qualitative urine analyzer 1 with the images of the urine sample captured at each of the imaging locations, stores them in the storage unit 36, and ends the imaging process shown in FIG. 7.

After the imaging process ends, the control component 26 controls the communication component 25 to send the captured images to the user terminal 50 through the communication unit 39. This allows the specialized clinical laboratory technician using the user terminal 50 to classify the material components included in the urine sample. At the same time, since the images of the urine sample are stored in the storage unit 36, the clinical laboratory technician can later check the images that were captured in the urine material component analyzer 3.

It will be noted that when the reference imaging locations are set in step S10, it is preferred that each of the reference imaging locations be set so that the range of the imaging plane 18 when the imaging plane 18 is viewed along the Z-axis direction includes the center portion of the cell 5. The "range of the imaging plane 18 when the imaging plane 18 is viewed along the Z-axis direction" is within the range of the projection plane obtained by projecting the imaging plane 18 onto the moving plane. The "center portion of the cell 5" refers to a region within a predetermined range from the center point of the hollow portion 13 of the cell 5 when the cell 5 is viewed along the Z-axis direction and is a region that does not touch the boundary of the hollow portion 13.

FIG. 10 is a drawing showing an example of the imaging plane 18 as viewed along the Z-axis direction. In the example shown in FIG. 10, the center portion of the cell 5 is included inside the imaging plane 18. Material components included in the urine sample are more likely to gather in the center portion of the cell 5 compared with other places. Consequently, as shown in FIG. 10 for example, it is preferred to set the reference imaging locations so that the imaging plane 18 as viewed along the Z-axis direction includes the center portion of the cell 5. In this case, the probability is higher that material components will be included in the images captured at the interpolated imaging locations compared with setting the reference imaging locations so that the imaging plane 18 as viewed along the Z-axis direction does not include the center portion of the cell 5.

Furthermore, although in the imaging process shown in FIG. 7 a triangle generated by interconnecting the reference points at each of the reference imaging locations such as shown in FIG. 9 is generated as the imaging plane 18, the method of generating the imaging plane 18 is not limited to this. For example, the calculation component 23 may perform linear interpolation on the reference points and generate a plane passing through each of the reference points as the imaging plane 18A.

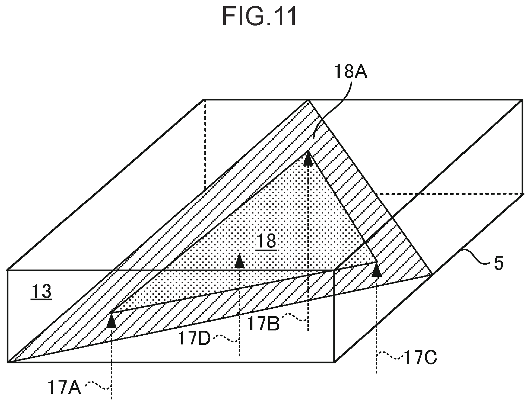

FIG. 11 is a drawing showing an example of an imaging plane 18A obtained by extending side edge portions of the imaging plane 18 shown in FIG. 9 and which passes through the reference points at each of the reference imaging locations. When the imaging plane 18A such as shown in FIG. 11 is generated in step S90 of FIG. 7, in step S100 of FIG. 7 the control component 26 moves the interpolated imaging location within the range of a projection plane obtained by projecting the imaging plane 18A onto the moving plane.

It will be noted that the specified number of reference imaging locations may be two. In this case, in step S90 of FIG. 7, instead of the imaging plane 18, an imaging line (not shown in the drawings) interconnecting the two reference imaging locations is generated by interpolating the reference points of the two reference imaging locations with a straight line. When the imaging line is generated, in step S100 of FIG. 7 the control component 26 moves the imaging location of the imaging device 40 along the imaging line. In step S110 of FIG. 7, the calculation component 23 assigns the moving plane coordinate values at the destination interpolated imaging location to the interpolation equation representing the imaging line and calculates the position of the imaging line at the destination interpolated imaging location. Consequently, the control component 26 captures an image focused on the position of the imaging line.

Furthermore, although in the imaging process shown in FIG. 7 the imaging plane 18 is represented by a flat plane, the area between the reference points at each of the reference imaging locations may be interpolated using Lagrange interpolation or spline interpolation, for example, and the imaging plane 18 may be represented by a combination of flat planes or a curved plane.

In this way, the urine material component analyzer 3 pertaining to this embodiment searches for a reference point where the material components are in focus while changing the focus position from the lower surface of the cell 5 in the upward direction only at the reference imaging locations and generates the imaging plane 18 by interpolating between the reference points. Furthermore, the urine material component analyzer 3 captures an image by focusing the imaging device 40 on the position of the imaging plane 18 from the start at the interpolated imaging location. Consequently, the amount of time required to image a urine sample can be shortened compared with capturing images while searching for the height in the cell 5 at which material components in the urine are present at all imaging locations.

### <Example Modification of Imaging Process>

In the imaging process shown in FIG. 7, the images captured at the interpolated imaging locations are images focused on the position of the imaging plane 18, i.e., imaging plane images, but material components do not always appear in the imaging plane images. The speed at which the material components settle is dependent on the sizes of the material components, so although crystals, for example, tend to settle quickly, red blood cells tend to settle more slowly than crystals, and bacteria tend to float in urine samples without settling. For that reason, the heights at which material components are present may differ depending on the type of the formed element.

Hereinafter, an imaging process for capturing images focused on a plurality of heights based on the position of the imaging plane 18 will be described.

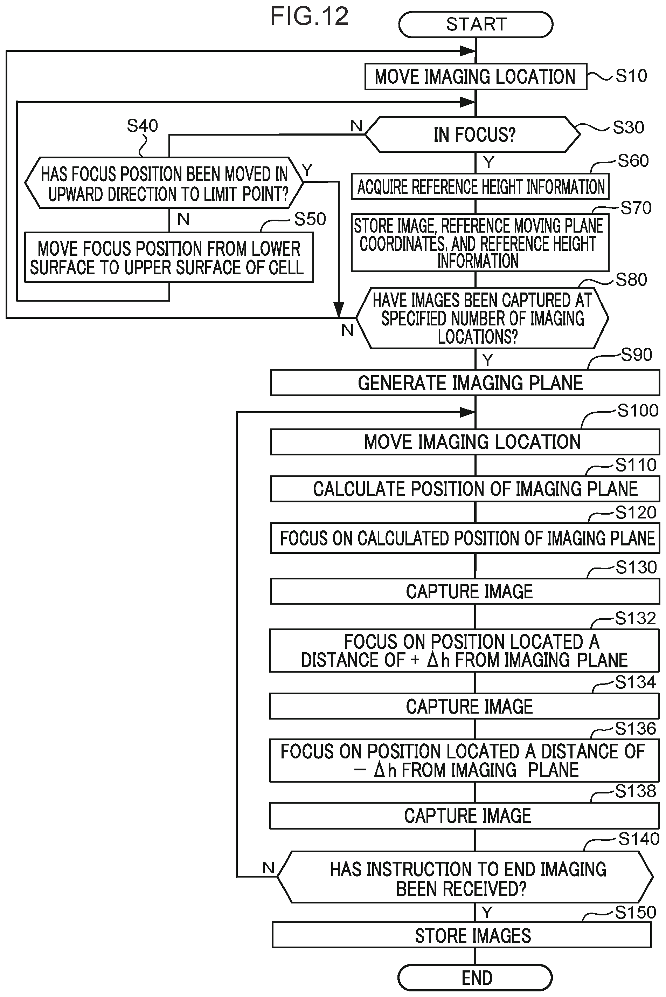

FIG. 12 is a flowchart showing an example of the flow of an imaging process for imaging material components included in a urine sample, which is executed by the CPU 31 of the urine material component analyzer 3 when it receives the sample ID of the urine sample from the qualitative urine analyzer 1. The CPU 31 of the urine material component analyzer 3 reads the imaging program 36B stored in the storage unit 36 and executes the imaging process.

The imaging process shown in FIG. 12 differs from the imaging process shown in FIG. 7 in that steps S132 to S138 are added, but other processes are the same as those of the imaging process shown in FIG. 7. Consequently, hereafter, the imaging process shown in FIG. 12 will be described focusing on the processes of steps S132 to S138.

After the control component 26 captures the imaging plane image in step S130 of FIG. 12, it moves to step S132.

In step S132, the control component 26 controls the imaging device 40 to focus the imaging device 40 on a position moved Δh in the upward direction along the Z-axis direction from the position of the imaging plane 18 that was calculated in step S110. That is, the control component 26 controls the imaging device 40 to focus the imaging device 40 on a position located a distance of + Δh from the position of the imaging plane 18 at the interpolated imaging location. The distance Δh is, for example, a value that is stored in advance in the storage unit 36 and changeable by the clinical laboratory technician.

In step S134, the control component 26 controls the imaging device 40 to capture an image in which the imaging device 40 is focused on the position located the distance of + Δh from the position of the imaging plane 18, i.e., an upper image. Because of this, it becomes easier for floating material components and material components that settle at a slower rate than the material components captured at the reference imaging locations to be captured compared with capturing just the imaging plane image at the interpolated imaging location.

In step S136, the control component 26 controls the imaging device 40 to focus the imaging device 40 on a position moved Δh in the downward direction along the Z-axis direction from the position of the imaging plane 18 that was calculated in step S110. That is, the control component 26 controls the imaging device 40 to focus the imaging device 40 on a position located a distance of - Δh from the position of the imaging plane 18 at the interpolated imaging location.

In step S138, the control component 26 controls the imaging device 40 to capture an image in which the imaging device 40 is focused on the position located the distance of - Δh from the position of the imaging plane 18, i.e., a lower image. Because of this, it becomes easier for material components that settle at a faster rate than the material components captured at the reference imaging locations to be captured compared with capturing just the imaging plane image at the interpolated imaging location.

It will be noted that when the images captured at the interpolated imaging locations are stored in the storage unit 36 by the process of step S150, the control component 26 stores the images captured at each of the interpolated imaging locations in the storage unit 36 so that imaging plane images, upper images, and lower images can be distinguished.

In this way, the urine material component analyzer 3 captures the imaging plane image, the upper image, and the lower image at each of the interpolated imaging locations by focusing the imaging device 40 on the position of the imaging plane 18 and on positions shifted Δh in the upward direction and the downward direction from the imaging plane 18. Consequently, more material components may be able to be captured compared with the imaging process shown in FIG. 7.

It will be noted that although in the above description the upper image and the lower image were captured by focusing the imaging device 40 on positions located distances of ± Δh from the position of the imaging plane 18, the images may be captured by focusing the imaging device 40 on positions located distances of ± kΔh from the position of the imaging plane 18. Index k is an integer equal to or greater than 1. That is, the urine material component analyzer 3 may capture pluralities of upper images and lower images at the same interpolated imaging location. Furthermore, the urine material component analyzer 3 need not invariably capture the same number of upper images and lower images, and, for example, may capture one lower image and two upper images. Moreover, the urine material component analyzer 3 may, for example, capture just either one of the upper image or the lower image.

Moreover, the urine material component analyzer 3 may capture at least one of the upper image and the lower image in addition to the imaging plane image also at the reference imaging locations.

### <Image Combination>

The urine material component analyzer 3 may transmit as is to the user terminal 50 the images captured at each of the reference imaging locations and the interpolated imaging locations, but it may also generate a panoramic image from the captured images and transmit the panoramic image of the urine sample to the user terminal 50.

The control component 26 references the moving plane coordinate values and the height information of the imaging locations associated with the images and acquires from the storage unit 36 multiple images captured at adjacent imaging locations. "Adjacent imaging locations" refers to imaging locations in which the distance between them as calculated from the moving plane coordinate values associated with the images is the shortest.

The control component 26 removes overlapping portions of the acquired multiple images that overlap images of adjacent imaging locations and joins the remaining portions together to generate a panoramic image of the urine sample that captures a wider range than the imaging ranges of each of the images. Since a panoramic image has a wider checkable range, the accuracy with which the material components are classified is improved compared with classifying the material components by referencing the images captured at each of the imaging locations.

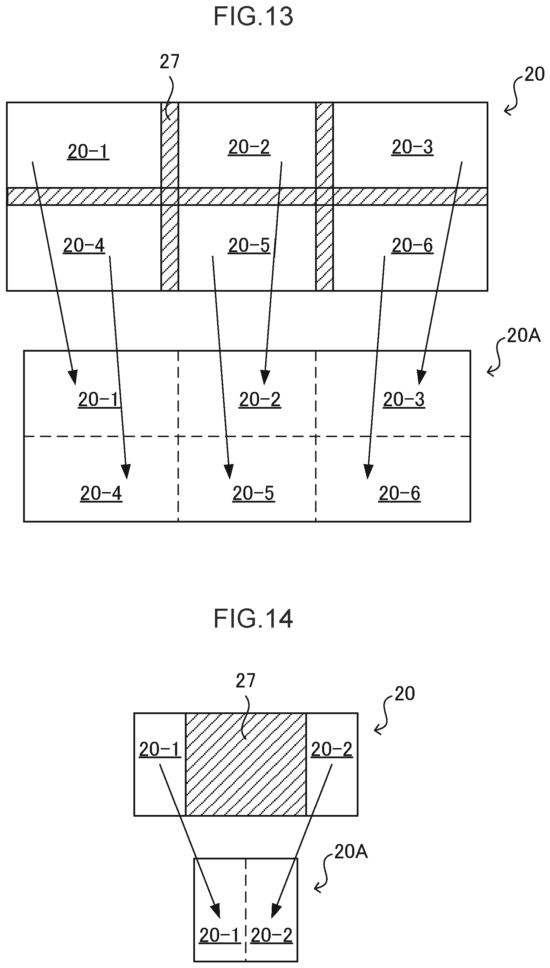

FIG. 13 is a drawing showing an example of panoramic image generation in the urine material component analyzer 3. Images 20 in FIG. 13 represent the images captured at each of the imaging locations.

FIG. 13 shows an example where overlapping portions 27 of six images from image 20-1 to image 20-6 whose imaging locations are adjacent are removed and the remaining image portions are joined together to generate one panoramic image 20A.

When generating a panoramic image using the images captured by the imaging process shown in FIG. 12, the control component 26 generates the panoramic image using images of the same type out of the images captured at adjacent imaging locations. Examples of image types include the imaging plane images, the lower images, and the upper images.

It will be noted that because the optical axis of the imaging device 40 passes through the centers of the images, the side edge portions of the images have a lower degree of focus consistency compared with the center portions of the images. Consequently, it is preferred that the control component 26 control the imaging locations so that each of the images configuring the panoramic image includes the center portions of the images.

FIG. 14 is a drawing showing an example of a panoramic image configured by side edge portions of images. In the example shown in FIG. 14, the overlapping portion 27 between the image 20-1 and the image 20-2 whose imaging locations are adjacent extends to the center portions of each of the image 20-1 and the image 20-2. Consequently, the panoramic image 20A, generated by removing the overlapping portion 27 and joining together the remaining image portions, is configured by the side edge portions of the image 20-1 and the image 20-2. As a result, in the panoramic image 20A shown in FIG. 14, the shapes of the material components included in the panoramic image 20A end up being more blurred, and it becomes more difficult to classify the material components, than in a panoramic image configured from images including the center portions of the same image 20-1 and image 20-2.

Needless to say, the method of imaging material components included in urine samples in the urine material component analyzer 3 described above can be used to image material components not only in urine but also in blood, cells, and body fluids.

Although a configuration of the urine material component analyzer 3 has been described above, the disclosed configuration of the urine material component analyzer 3 is only an example, and the configuration of the urine material component analyzer 3 is not limited to the scope described in the embodiment. Various modifications or improvements can be made to the embodiment without departing from the spirit of this disclosure, and the technical scope of the disclosure also includes configurations to which such modifications or improvements have been made.

For example, the order of internal processes in the imaging processes shown in FIG. 7 and FIG. 12 may be changed without departing from the spirit of this disclosure.

Furthermore, in the above embodiment, as an example, a configuration where the imaging processes are realized with software was described. However, processes equivalent to those in the imaging process flowcharts shown in FIG. 7 and FIG. 12 may be performed by hardware. In this case, the speed of the processes is increased compared with realizing the imaging processes with software.

In the above embodiment, "processor" refers to processors in a broad sense and includes general-purpose processors (e.g., the CPU 31) and dedicated processors (e.g., graphics processing units, or GPUs, application-specific integrated circuits, or ASICs, field-programmable gate arrays, or FPGAs, and programmable logic devices).

The operations of the processor in the above embodiment need not be performed by one processor and may be performed by multiple processors in physically separate locations working together. Furthermore, the order of the operations of the processor is not limited to just the order described in the above embodiment and may be changed as appropriate.

Furthermore, in the above embodiment, an example where the imaging programs 36A, 36B are stored in advance in the storage unit 36 was described. However, the storage destination of the imaging programs 36A, 36B is not limited to the storage unit 36. The imaging programs 36A, 36B of this disclosure can also be provided in a form in which they are recorded in storage media readable by the computer 30.

For example, the imaging programs 36A, 36B may be provided in a form in which they are recorded in an optical disc such as a compact disk read-only memory (CD-ROM), a digital versatile disk read-only memory (DVD-ROM), or a Blu-ray disc. Furthermore, the imaging programs 36A, 36B may be provided in a form in which they are recorded in a portable semiconductor memory such as a USB memory or a memory card. The storage unit 36, CD-ROM, DVD-ROM, Blu-ray disc, USB memory, and memory card are examples of a non-transitory storage medium.

Moreover, the urine material component analyzer 3 may download the imaging programs 36A, 36B through the communication unit 39 from a file server or the like (not shown in the drawings) connected to the communication line 4 and store the downloaded imaging programs 36A, 36B in the storage unit 36 of the urine material component analyzer 3. In this case, the CPU 31 of the urine material component analyzer 3 reads from the storage unit 36 the imaging programs 36A, 36B downloaded from the file server or the like and executes the imaging processes. It will be noted that this disclosure can also be applied to programs and program products.

The disclosure of Japanese Patent Application No. 2023-186985, filed on October 31, 2023, is incorporated in its entirety by reference herein. All documents, patent applications, and technical standards mentioned in this specification are incorporated herein by reference to the same extent as if each individual document, patent application, or technical standard were specifically and individually indicated to be incorporated by reference.

Supplementary notes pertaining to this disclosure are described below.

### (Supplementary Note 1)

An imaging method in which a computer executes a processing to:
move, along a moving plane intersecting a direction of gravity, an imaging location of an imaging device that images, along the direction of gravity, a cell having a hollow portion in which a urine sample is enclosed and acquire, at a plurality of imaging locations, reference height information representing a position, in the direction of gravity in the cell, of material components included in the urine sample in a case in which the material components are in focus and reference imaging location information representing imaging locations of the material components imaged by the imaging device using coordinate values of two-dimensional coordinates provided in the moving plane;
calculate, by interpolation using the reference height information and the reference imaging location information at the plurality of imaging locations, positions of the material components at other imaging locations different from the plurality of imaging locations; and
control the imaging device to focus on calculated positions of the material components and capture sample images that are images of the urine sample at the other imaging locations.

### (Supplementary Note 2)

The imaging method of supplementary note 1, wherein the processing
sets at least three imaging locations as the plurality of imaging locations,
generates an imaging plane obtained by calculating, by interpolation using the reference height information and the reference imaging location information at the plurality of imaging locations, positions of the material components at other imaging locations different from the plurality of imaging locations, the imaging plane contacting the material components located at positions represented by the reference height information captured at the plurality of imaging locations, and
controls the imaging device to focus on a position of the imaging plane represented by intersections between straight lines passing through the other imaging locations in the direction of gravity and the generated imaging plane and captures the sample images at the other imaging locations.

### (Supplementary Note 3)

The imaging method of supplementary note 2, wherein the processing controls the imaging device to shift, by a predetermined distance along the direction of gravity from the position of the imaging plane corresponding to each of the other imaging locations, a focus position at each of the other imaging locations and captures, at each of the other imaging locations, the sample images focused on the position of the imaging plane and the sample images focused on positions shifted the predetermined distance along the direction of gravity from the imaging plane.

### (Supplementary Note 4)

The imaging method of supplementary note 2 or supplementary note 3, wherein the processing sets the plurality of imaging locations so that a center portion of the cell is included inside the imaging plane as viewed along the direction of gravity.

### (Supplementary Note 5)

The imaging method of any one of supplementary note 1 to supplementary note 4, wherein the processing removes overlapping portions included in each of the sample images captured at adjacent imaging locations and joins together the remaining portions to generate a panoramic image of the urine sample that captures a wider range than the imaging ranges of the images in the imaging device.

### (Supplementary Note 6)

The imaging method of supplementary note 5, wherein the processing controls the imaging location of the imaging device so that the remaining portions include center portions of the sample images.

### (Supplementary Note 7)

An imaging program for causing a computer to execute a processing to:
move, along a moving plane intersecting the direction of gravity, an imaging location of an imaging device that images, along the direction of gravity, a cell having a hollow portion in which a urine sample is enclosed and acquire, at a plurality of imaging locations, reference height information representing a position, in the direction of gravity in the cell, of material components included in the urine sample in a case in which the material components are in focus and reference imaging location information representing imaging locations of the material components imaged by the imaging device using coordinate values of two-dimensional coordinates provided in the moving plane;
calculate, by interpolation using the reference height information and the reference imaging location information at the plurality of imaging locations, positions of the material components at other imaging locations different from the plurality of imaging locations; and
control the imaging device to focus on calculated positions of the material components and capture sample images that are images of the urine sample at the other imaging locations.

### (Supplementary Note 8)

A non-transitory storage medium storing a program executable by a computer to execute an imaging process, the image process including:
an acquisition step of moving, along a moving plane intersecting the direction of gravity, an imaging location of an imaging device that images, along the direction of gravity, a cell having a hollow portion in which a urine sample is enclosed and acquiring, at a plurality of imaging locations, reference height information representing a position, in the direction of gravity in the cell, of material components included in the urine sample when the material components are in focus and reference imaging location information representing imaging locations of the material components imaged by the imaging device using coordinate values of two-dimensional coordinates provided in the moving plane;
a calculation step of calculating, by interpolation using the reference height information and the reference imaging location information at the plurality of imaging locations, positions of the material components at other imaging locations different from the plurality of imaging locations; and
an imaging step of controlling the imaging device to focus on the calculated positions of the material components and capture sample images that are images of the urine sample at the other imaging locations.

### (Supplementary Note 9)

A computer program product including an imaging program causing a computer to execute a processing to:
move, along a moving plane intersecting the direction of gravity, an imaging location of an imaging device that images, along the direction of gravity, a cell having a hollow portion in which a urine sample is enclosed and acquire, at a plurality of imaging locations, reference height information representing a position, in the direction of gravity in the cell, of material components included in the urine sample in a case in which the material components are in focus and reference imaging location information representing imaging locations of the material components imaged by the imaging device using coordinate values of two-dimensional coordinates provided in the moving plane;
calculate, by interpolation using the reference height information and the reference imaging location information at the plurality of imaging locations, positions of the material components at other imaging locations different from the plurality of imaging locations; and
control the imaging device to focus on calculated positions of the material components and capture sample images that are images of the urine sample at the other imaging locations.

According to supplementary note 1, supplementary note 7, supplementary note 8, and supplementary note 9, there is the advantageous effect that, compared with capturing an image while focusing on material components included in a urine sample at each imaging location of a cell in which the urine sample is enclosed, the amount of time required to image the material components can be shortened.

According to supplementary note 2, there is the advantageous effect that the imaging location can be moved in the range of the generated imaging plane.

According to supplementary note 3, there is the advantageous effect that, compared with imaging just the position of the imaging plane, more material components can be imaged.

According to supplementary note 4, there is the advantageous effect that, compared with setting the plurality of imaging locations so that the imaging plane does not include the center portion of the cell 5, the probability that the images captured at the other imaging locations will include material components is higher.

According to supplementary note 5, there is the advantageous effect that, compared with classifying the material components by referring to the images captured at each imaging location, the accuracy with which the material components included in the urine sample are classified is improved.

According to supplementary note 6, there is the advantageous effect that, compared with classifying the material components by referring to a panoramic image configured from side edge portions of the images, the accuracy with which the material components included in the urine sample are classified is improved.

## Claims

1. An imaging method in which a computer executes processing to:
move, along a moving plane intersecting a direction of gravity, an imaging location of an imaging device that images, along the direction of gravity, a cell having a hollow portion in which a urine sample is enclosed, and acquire, at a plurality of imaging locations, reference height information representing a position, in the direction of gravity in the cell, of material components included in the urine sample in a case in which the material components are in focus, and reference imaging location information representing imaging locations of the material components imaged by the imaging device using coordinate values of two-dimensional coordinates provided in the moving plane;
calculate, by interpolation using the reference height information and the reference imaging location information at the plurality of imaging locations, positions of the material components at other imaging locations different from the plurality of imaging locations; and
control the imaging device to focus on calculated positions of the material components and capture sample images that are images of the urine sample at the other imaging locations.

2. The imaging method of claim 1, wherein the processing
sets at least three imaging locations as the plurality of imaging locations,
generates an imaging plane obtained by calculating, by interpolation using the reference height information and the reference imaging location information at the plurality of imaging locations, positions of the material components at other imaging locations different from the plurality of imaging locations, the imaging plane contacting the material components located at positions represented by the reference height information captured at the plurality of imaging locations, and
controls the imaging device to focus on a position of the imaging plane represented by intersections between straight lines passing through the other imaging locations in the direction of gravity and the generated imaging plane, and captures the sample images at the other imaging locations.

3. The imaging method of claim 2, wherein the processing
controls the imaging device to shift, by a predetermined distance along the direction of gravity from the position of the imaging plane corresponding to each of the other imaging locations, a focus position at each of the other imaging locations, and captures, at each of the other imaging locations, the sample images focused on the position of the imaging plane and the sample images focused on positions shifted the predetermined distance along the direction of gravity from the imaging plane.

4. The imaging method of claim 3, wherein the processing sets the plurality of imaging locations so that a center portion of the cell is included inside the imaging plane as viewed along the direction of gravity.

5. The imaging method of any one of claim 1 to claim 4, wherein the processing removes overlapping portions included in each of the sample images captured at adjacent imaging locations and joins together remaining portions to generate a panoramic image of the urine sample that captures a wider range than imaging ranges of images in the imaging device.

6. The imaging method of claim 5, wherein the processing controls the imaging location of the imaging device so that the remaining portions include center portions of the sample images.

7. An imaging program for causing a computer to execute processing to:
move, along a moving plane intersecting a direction of gravity, an imaging location of an imaging device that images, along the direction of gravity, a cell having a hollow portion in which a urine sample is enclosed, and acquire, at a plurality of imaging locations, reference height information representing a position, in the direction of gravity in the cell, of material components included in the urine sample in a case in which the material components are in focus, and reference imaging location information representing imaging locations of the material components imaged by the imaging device using coordinate values of two-dimensional coordinates provided in the moving plane;
calculate, by interpolation using the reference height information and the reference imaging location information at the plurality of imaging locations, positions of the material components at other imaging locations different from the plurality of imaging locations; and
control the imaging device to focus on calculated positions of the material components and capture sample images that are images of the urine sample at the other imaging locations.
